# EUROPEAN PATENT APPLICATION

(11) **EP 2 832 305 A1**
(43) Date of publication of application: **04.02.2015**
(21) Application number: 13768935.2
(22) Date of filing: 11.03.2013
(51) Int. Cl.: A61B 17/04, A61B 17/00, A61B 17/42

(54) **PUNCTURE INSTRUMENT AND PUNCTURE DEVICE**

(30) Priority: 30.03.2012 JP 2012079036
(71) Applicant: Terumo Kabushiki Kaisha, Tokyo 151-0072 (JP)
(72) Inventor: KAWAURA, Masakatsu, Ashigarakami-gun Kanagawa 259-0151 (JP); ARIURA, Shigeki, Fujinomiya-shi Shizuoka 418-0015 (JP)
(74) Representative: Casalonga
(86) International application number: PCT/JP2013/056681
(87) International publication number: WO 2013/146220

(57) **Abstract**

A puncture apparatus 1 includes a puncture device 10 for puncturing a living body tissue, a urethral-insertion member 4 of a longitudinal shape for being inserted into a urethra, a vaginal insertion member 5 of a longitudinal shape for being inserted into a vagina, and a supporting member 2 which supports the urethral-insertion member 4, vaginal insertion member 5 and a puncture member 3 of the puncture device 10. The puncture device 10 includes a puncture member 3 having a puncture needle 31 for puncturing a living body tissue, a shaft portion 33 and a connection portion 32 for connecting the puncture needle 31 and the shaft portion 33 to each other, and an indwelling article assembly 8 positioned at a distal end of the puncture needle 31 and having a needle tip portion 82 for puncturing a living body tissue and a living body tissue supporting indwelling article 81. The puncture needle 31 has a form of a pipe and has a tubular portion 312. The living body tissue supporting indwelling article 81 is accommodated in the hollow portion 312.

## Description

### Technical Field

The present invention relates to a puncture device and a puncture apparatus.

### Background Art

If a person suffers from a urinary incontinence, specifically if a person suffers from a stress urinary incontinence, then urine leakage is caused by application of abdominal pressure during a normal exercise or by laughing, coughing, sneezing and the like. The cause of this may be, for example, that the pelvic floor muscle which is a muscle for supporting the urethra is loosened by birth or the like.

For the treatment of urinary incontinence, a surgical treatment is effective, in which there is used, for example, a belt-shaped living body tissue supporting indwelling article called "sling." The sling is indwelled inside the body and the urethra is supported by the sling (for example, refer to Patent Document 1). In order to indwell the sling inside the body, an operator would incise the vagina with a surgical knife, dissect a region between the urethra and the vagina, and communicate the dissected region and the outside with each other through an obturator foramen using a puncture needle or the like. Then, in such a state, the sling is indwelled into the body.

However, if the vagina is incised once, then there is the possibility that there may occur such a situation that the sling is exposed to the inside of the vagina from a wound caused by the incision thereof, and there is the possibility that complications may be caused by an infection from the wound or the like. Further, since the vagina is incised, there is such a defect that the invasion is great and the burden on the patient is heavy. Further, there is the possibility that the urethra or the like may be damaged in the course of the procedure by the operator, and also there is the possibility that the operator itself may be damaged at its fingertip.

### Prior Art Document

### Patent Document

Patent Document 1: Japanese Patent Laid-Open No. 2010-99499

### Summary of Invention

### Technical Problem

An object of the present invention is to provide a puncture device and a puncture apparatus by which a living body tissue supporting indwelling article can be implanted into a living body, in which the burden on the patient is light, the safety of the patient is high and also the safety of the operator is high.

### Technical Solution

Such an object as just described is achieved by the present invention described below.

The present invention provides a puncture device, including:
a puncture needle having a hollow portion;
a needle tip portion positioned at a distal end of the puncture needle and configured to puncture a living body tissue; and
an elongated living body tissue supporting indwelling article accommodated in the hollow portion of the puncture needle and configured to be implanted into a living body to support the living body tissue.

In the puncture device of the present invention, preferably the needle tip portion is removably held by or fixed to a distal end portion of the living body tissue supporting indwelling article.

In the puncture device of the present invention, preferably the living body tissue supporting indwelling article includes:
a belt-like element configured to attach to a living body tissue;
a first string-like element connected at a proximal end thereof to a distal end of the belt-like element;
a second string-like element connected at a distal end thereof to a proximal end of the belt-like element; and
a holding member connected to a proximal end of the second string-like element and removably held by a proximal end of the puncture needle;
the needle tip portion being removably held by or fixed to a distal end of the first string-like element.

In the puncture device of the present invention, preferably the needle tip portion has an anchoring effect of preventing the needle tip portion from returning to an opposite direction to a puncturing direction of the needle tip portion.

In the puncture device of the present invention, preferably the needle tip portion is fixedly mounted at or integrated with a distal end portion of the puncture needle.

In the puncture device of the present invention, preferably the puncture needle has a variable length.

In the puncture device of the present invention, preferably the puncture needle has a flattened shape as viewed in a longitudinal direction thereof, and
the living body tissue supporting indwelling article has a belt-like portion which is attached to a living body tissue.

Preferably, the puncture device of the present invention is configured such that the puncture needle has a portion curved along a longitudinal direction thereof.

Preferably, the puncture device of the present invention is configured such that the puncture device is installed for rotational movement.

According to the present invention, a puncture apparatus includes:
the puncture device of the present invention;
a urethral-insertion member of a longitudinal shape configured to be inserted into a urethra; and
restriction means for restricting, when the puncture device is rotationally moved to puncture a living body tissue, a positional relationship between the puncture device and the urethral-insertion member such that the needle tip portion passes at a farther-position side from the center of rotational movement of the puncture device than the urethral-insertion member.

Preferably the puncture apparatus of the present invention further includes:
a vaginal insertion member of a longitudinal shape configured to be inserted into a vagina, wherein
the restriction means restricts a positional relationship between the puncture device and the vaginal insertion member such that, when the puncture device is rotationally moved to puncture a living body tissue, the needle tip portion does not collide with the vaginal insertion member.

Preferably, the puncture apparatus of the present invention is configured such that the puncture device has a shaft portion which provides a rotational axis of the rotational moment, and
the restriction means includes a supporting member which supports the shaft portion for rotational movement and supports the urethral-insertion member and the vaginal insertion member.

In the puncture apparatus of the present invention, preferably at least one of the urethral-insertion member and the vaginal insertion member has provided thereon suction means capable of attracting a living body tissue thereto.

In the puncture apparatus of the present invention, preferably the positional relationship between the urethral-insertion member and the vaginal insertion member is variable.

### Advantageous Effect

With the present invention, it is possible to implant a living body tissue supporting indwelling article into a living body readily, and when the living body tissue supporting indwelling article is implanted, the burden on the patient is light and the safety of the patient is high. Also the safety of the operator is high.

Especially, where the puncture apparatus includes the restriction means for restricting the positional relation between the puncture device and the urethral-insertion member such that, when the puncture device rotationally moves to puncture the living body tissue, the needle tip portion passes the farther-position side from the center of rotational movement of the puncture device than the urethral-insertion member, for example, when the puncture apparatus is to be used for the treatment of woman's urinary incontinence, the urethral-insertion member of the puncture apparatus is inserted into a urethra, and the puncture device is rotationally moved so that the living body is punctured by the puncture device. Thereupon, since the needle tip portion passes the farther-position side from the center of rotational movement of the puncture device than the urethral-insertion member, the puncture needle can puncture the living body avoiding the urethra. Consequently, the puncture device can be prevented from puncturing the urethra. Further, it is possible to prevent a fingertip of the operator from being punctured by the puncture device.

Further, when the living body tissue supporting indwelling article for the treatment of urinary incontinence is to be implanted, no incision of the vaginal wall is required, and the living body tissue supporting indwelling article can be implanted by a low invasive manual procedure. Further, such a situation that, as in a case in which the vagina is incised, the living body tissue supporting indwelling article is exposed to the inside of the vagina through a wound caused by the incision or that such complications as an infection from the wound occur can be prevented. Therefore, the living body tissue supporting indwelling article can be implanted in very high safety and with certainty.

Further, since the living body tissue supporting indwelling article is accommodated in the hollow portion of the puncture needle, the living body tissue supporting indwelling article can be implanted into a living body readily.

### Brief Description of Drawings

[FIG. 1]

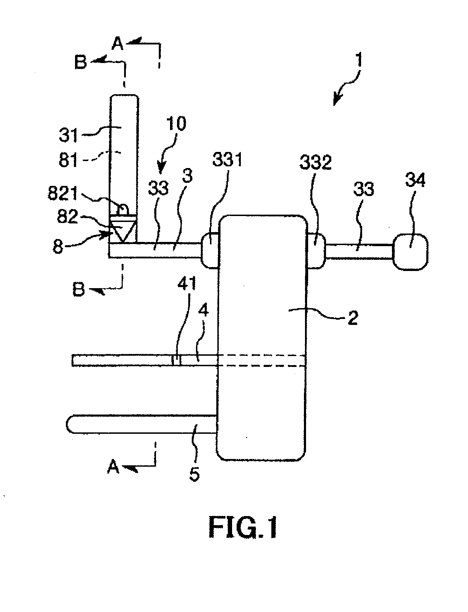

   FIG. 1 is a lateral view depicting a first embodiment of a puncture apparatus of the present invention.
[FIG. 2]

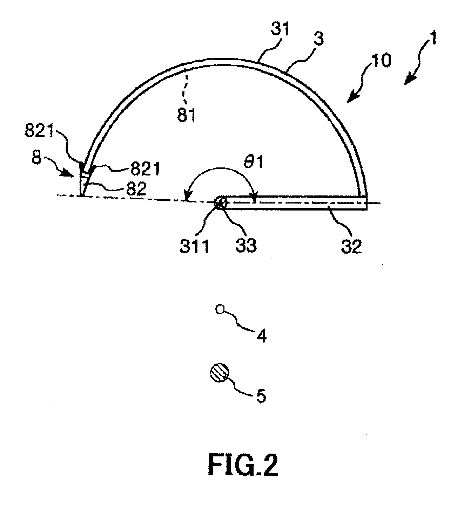

   FIG. 2 is a cross sectional view taken along line A-A in FIG. 1.
[FIG. 3]

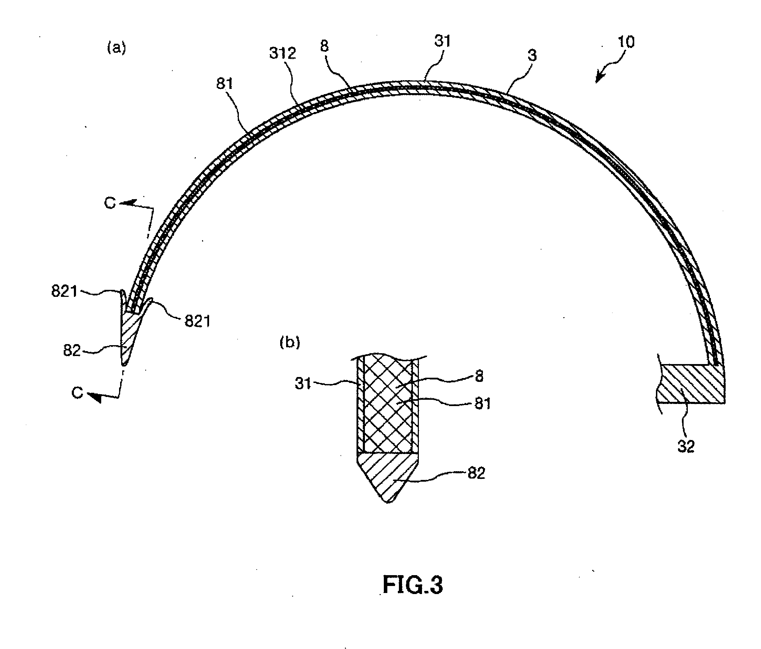

   FIG. 3 is a cross sectional view of a puncture device of the puncture apparatus depicted in FIG. 1.
[FIG. 4]

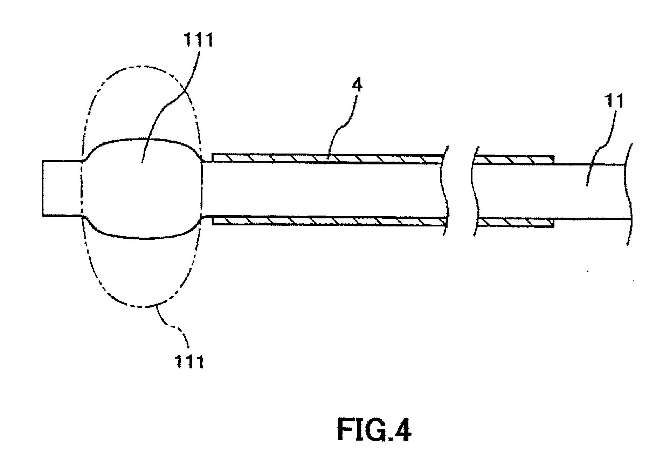

   FIG. 4 is a cross sectional view illustrating a state in which a balloon catheter is inserted in a urethral-insertion member of the puncture apparatus depicted in FIG. 1.
[FIG. 5]

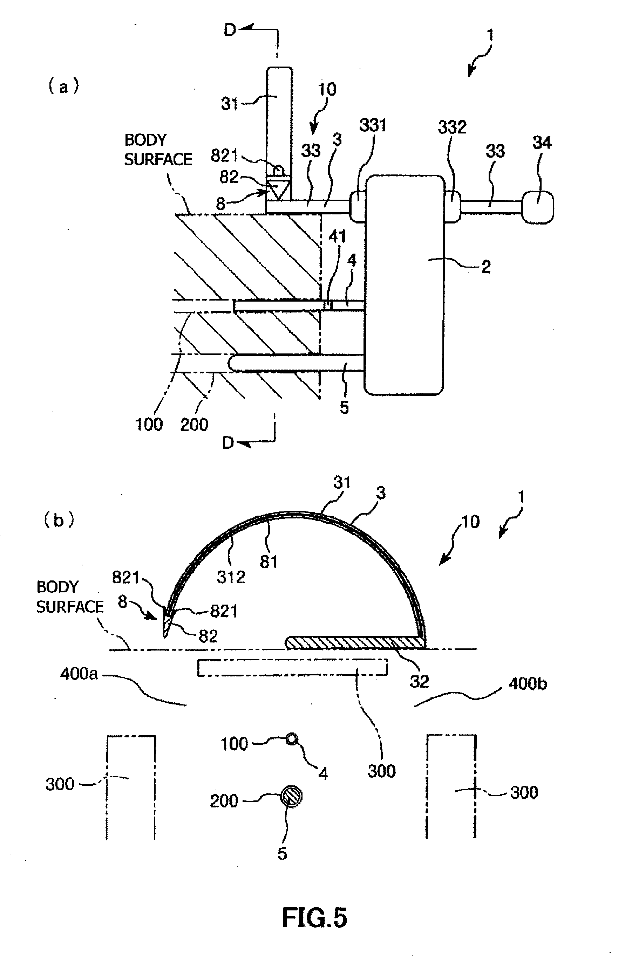

   FIG. 5 is a view illustrating an operation procedure of the puncture apparatus depicted in FIG. 1.
[FIG. 6]

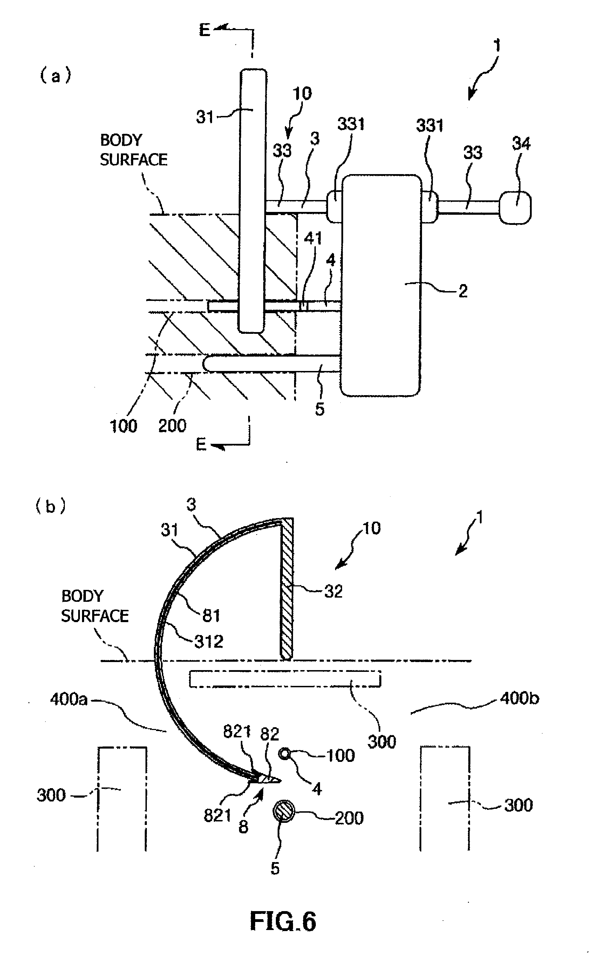

   FIG. 6 is a view illustrating an operation procedure of the puncture apparatus depicted in FIG. 1.
[FIG. 7]

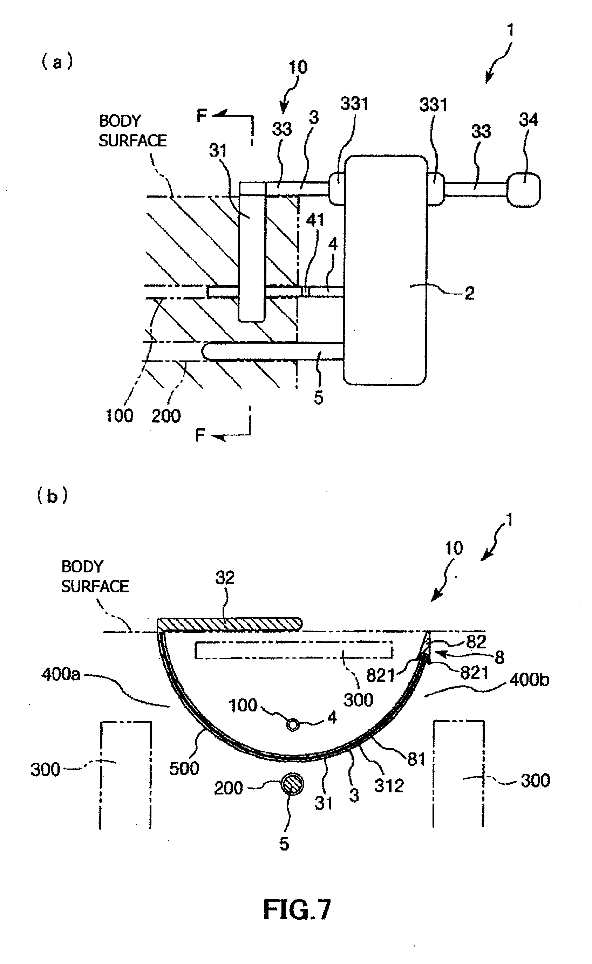

   FIG. 7 is a view illustrating an operation procedure of the puncture apparatus depicted in FIG. 1.
[FIG. 8]

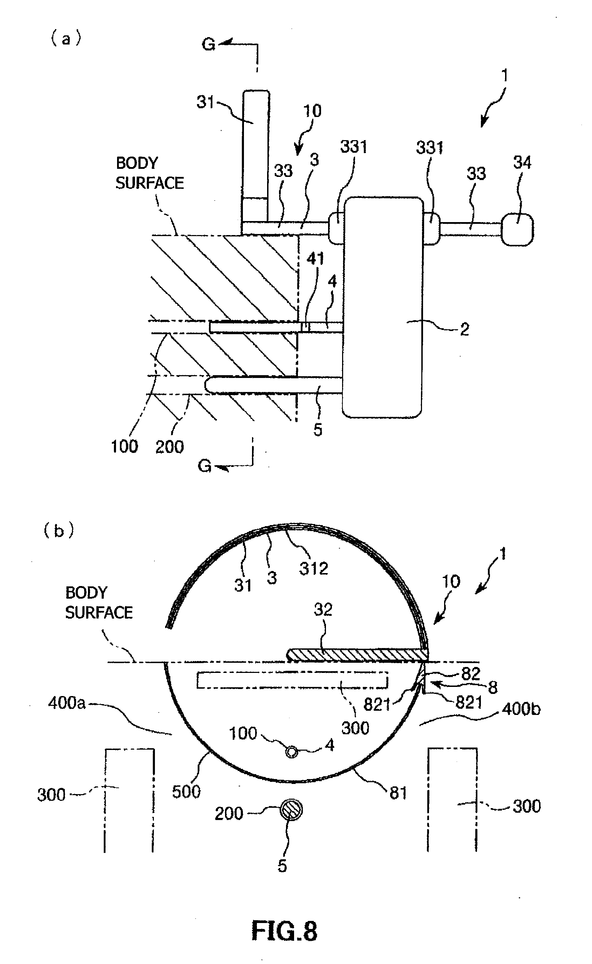

   FIG. 8 is a view illustrating an operation procedure of the puncture apparatus depicted in FIG. 1.
[FIG. 9]

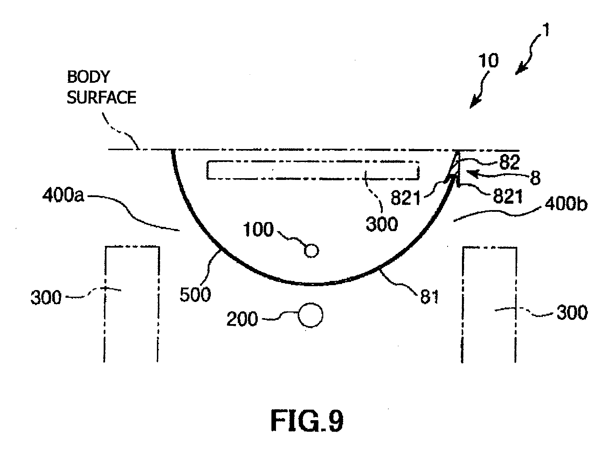

   FIG. 9 is a view illustrating an operation procedure of the puncture apparatus depicted in FIG. 1.
[FIG. 10]

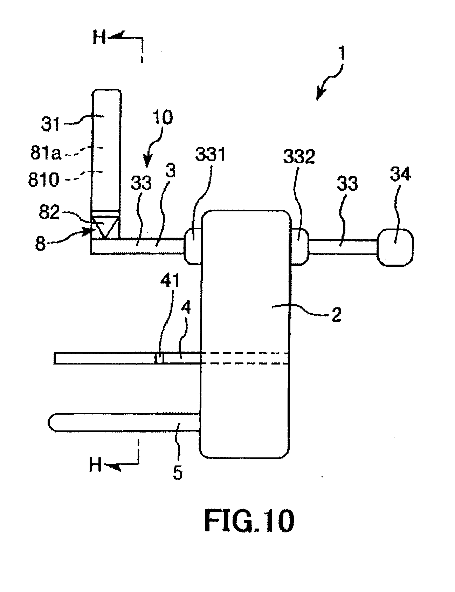

   FIG. 10 is a side elevational view depicting a second embodiment of the puncture apparatus of the present invention.
[FIG. 11]

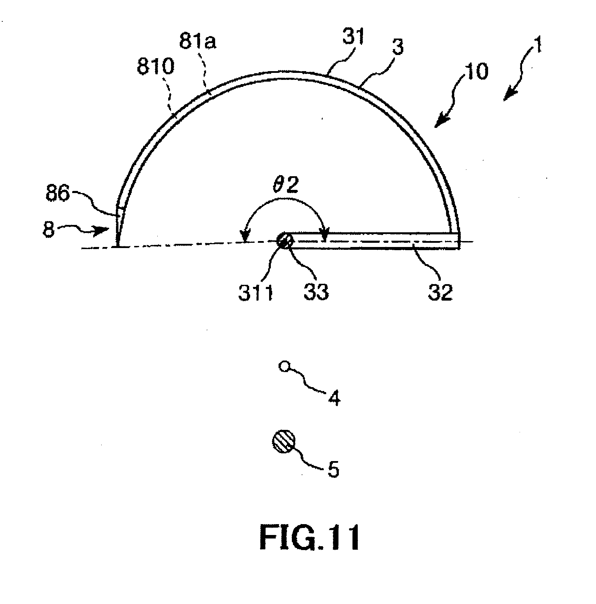

   FIG. 11 is a cross sectional view taken along line H-H in FIG. 10.
[FIG. 12]

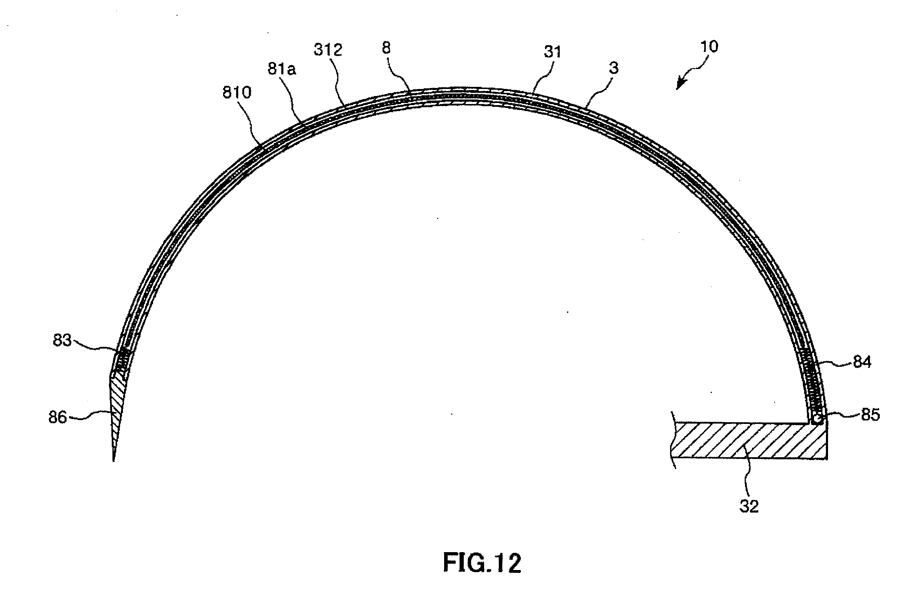

   FIG. 12 is a cross sectional view of a puncture device of the puncture apparatus depicted in FIG. 10.
[FIG. 13]

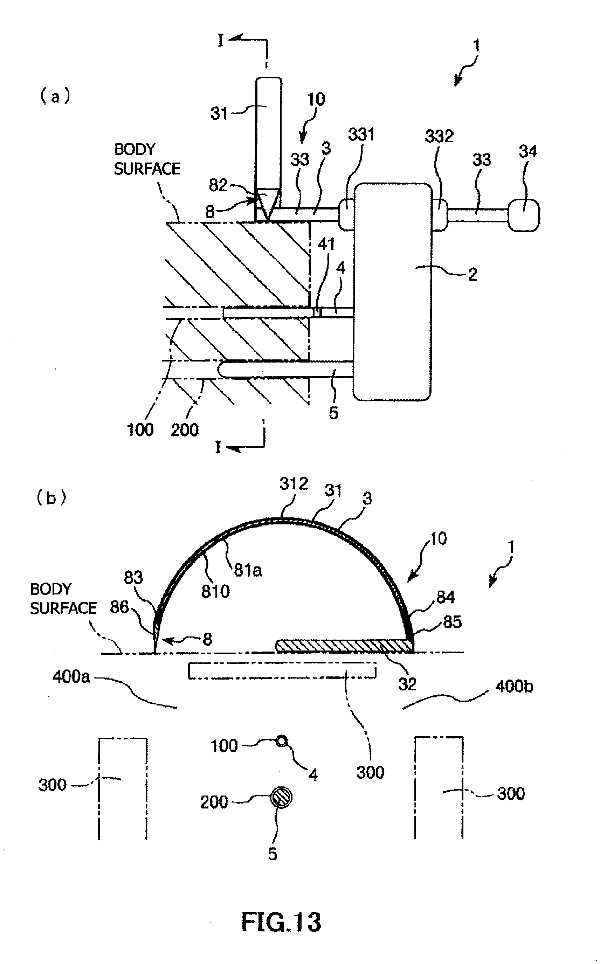

   FIG. 13 is a view illustrating an operation procedure of the puncture apparatus depicted in FIG. 10.
[FIG. 14]

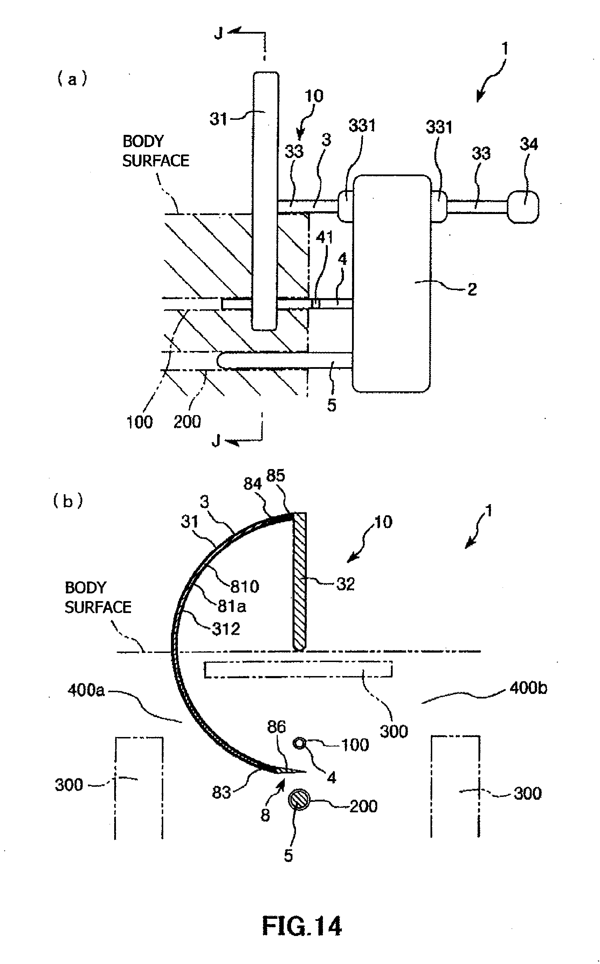

   FIG. 14 is a view illustrating an operation procedure of the puncture apparatus depicted in FIG. 10.
[FIG. 15]

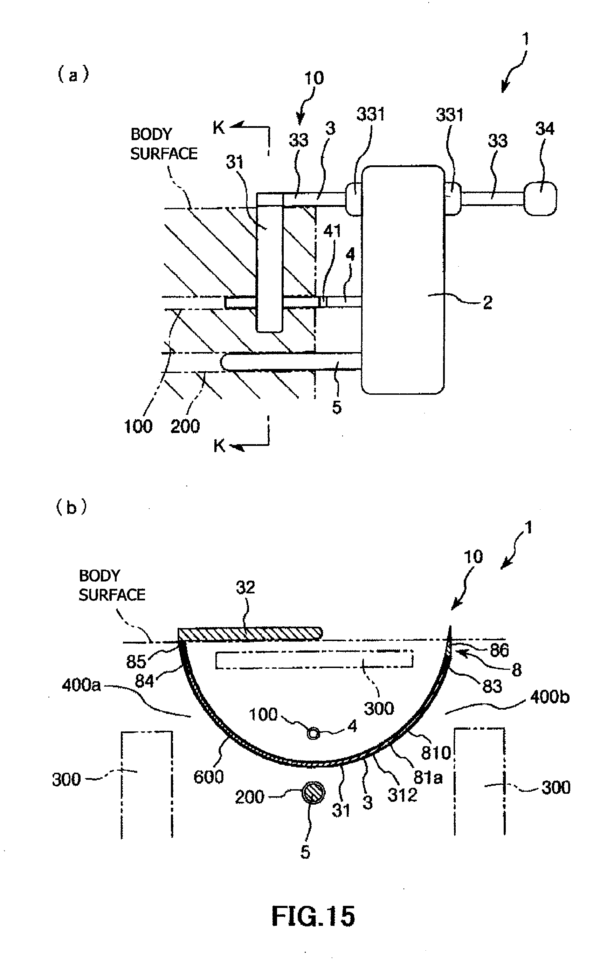

   FIG. 15 is a view illustrating an operation procedure of the puncture apparatus depicted in FIG. 10.
[FIG. 16]

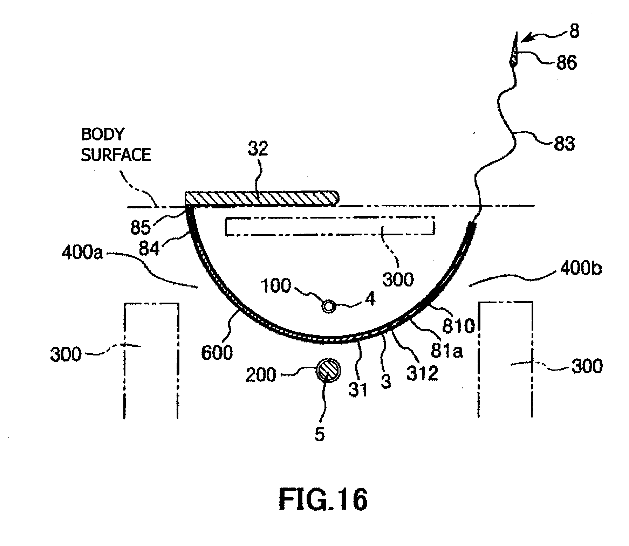

   FIG. 16 is a view illustrating an operation procedure of the puncture apparatus depicted in FIG. 10.
[FIG. 17]

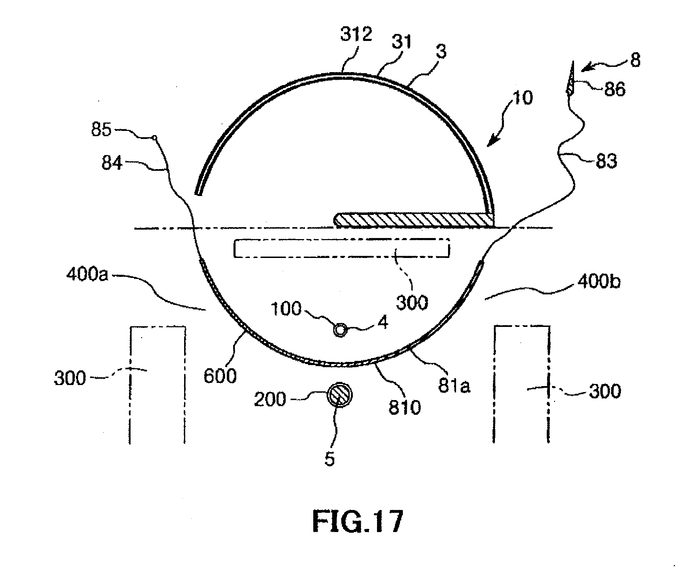

   FIG. 17 is a view illustrating an operation procedure of the puncture apparatus depicted in FIG. 10.
[FIG. 18]

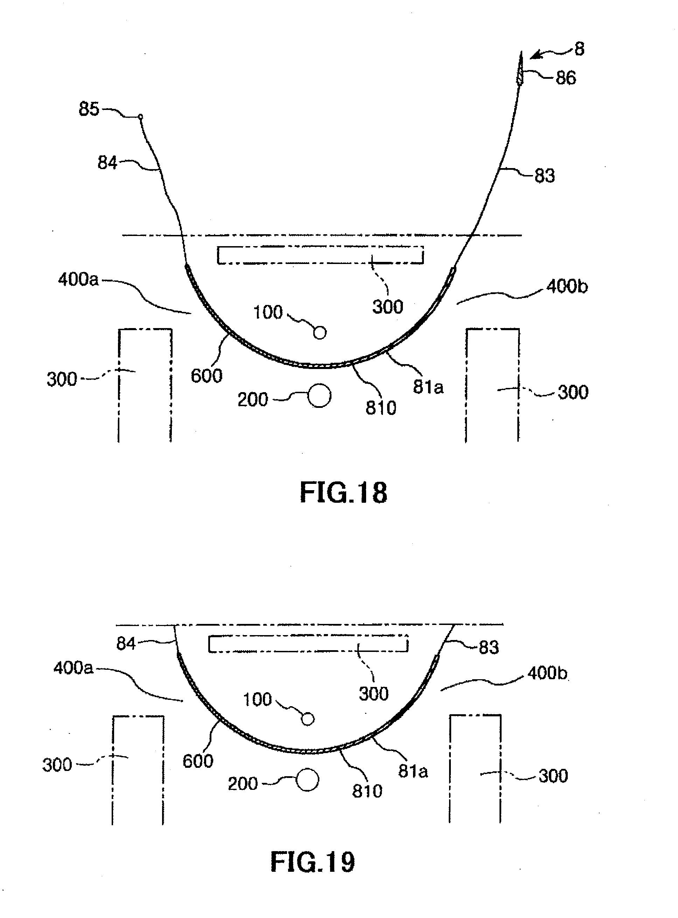

   FIG. 18 is a view illustrating an operation procedure of the puncture apparatus depicted in FIG. 10.
[FIG. 19]
   FIG. 19 is a view illustrating an operation procedure of the puncture apparatus depicted in FIG. 10.
[FIG. 20]

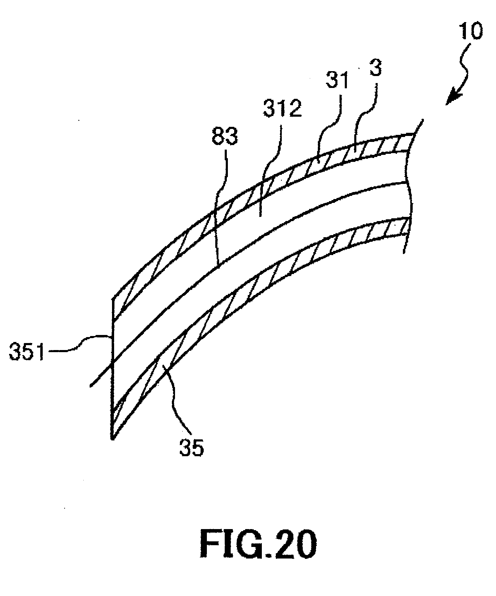

   FIG. 20 is a cross sectional view depicting a puncture device according to a third embodiment of the puncture apparatus of the present invention.
[FIG. 21]

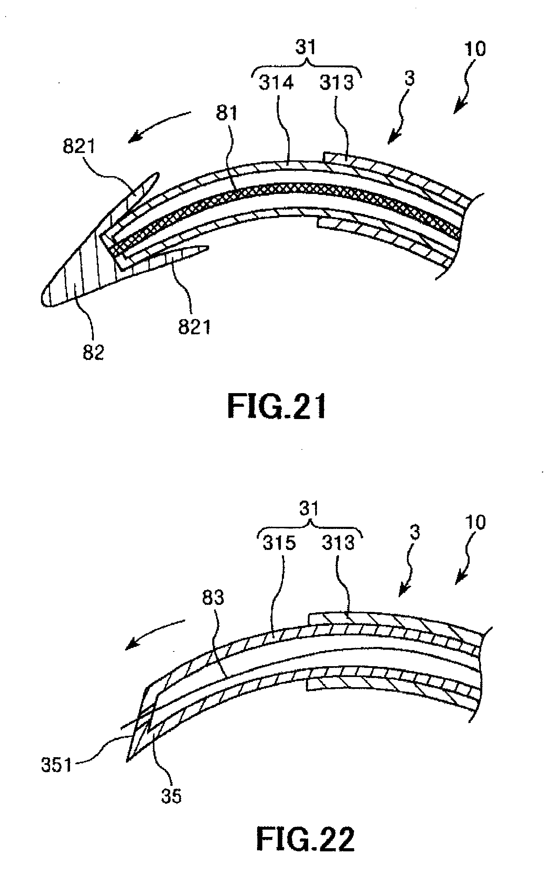

   FIG. 21 is a cross sectional view depicting a puncture device according to a fourth embodiment of the puncture apparatus of the present invention.
[FIG. 22]
   FIG. 22 is a cross sectional view depicting a puncture device according to a fifth embodiment of the puncture apparatus of the present invention.
[FIG. 23]

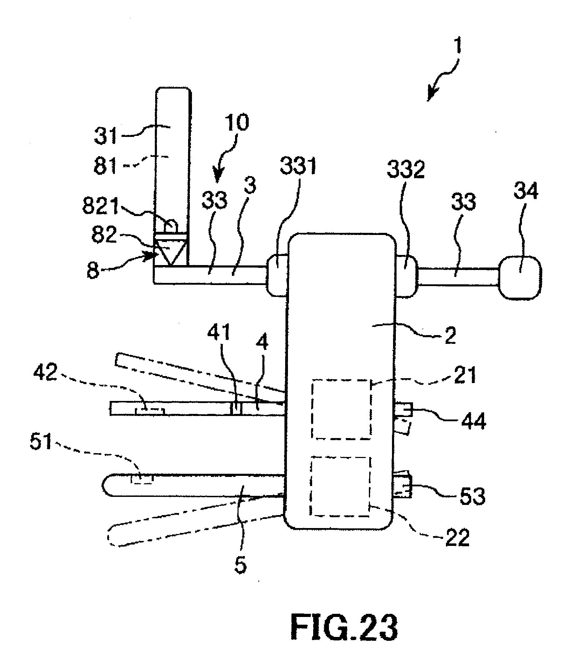

   FIG. 23 is a side elevational view depicting a sixth embodiment of the puncture apparatus of the present invention.
[FIG. 24]

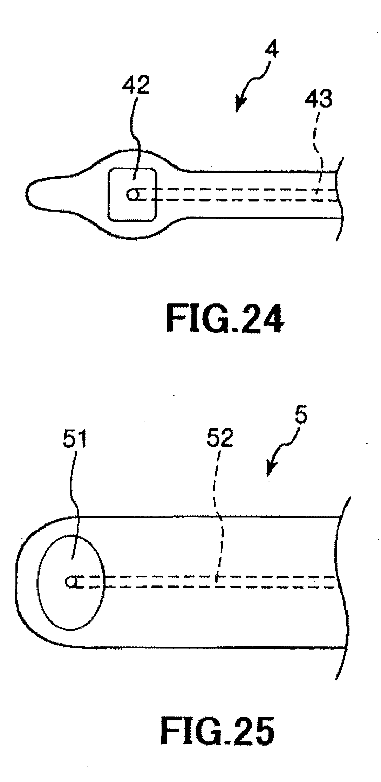

   FIG. 24 is a bottom plan view depicting a urethral-insertion member of the puncture apparatus depicted in FIG. 23.
[FIG. 25]
   FIG. 25 is a top plan view depicting a vaginal insertion member of the puncture apparatus depicted in FIG. 23.
[FIG. 26]

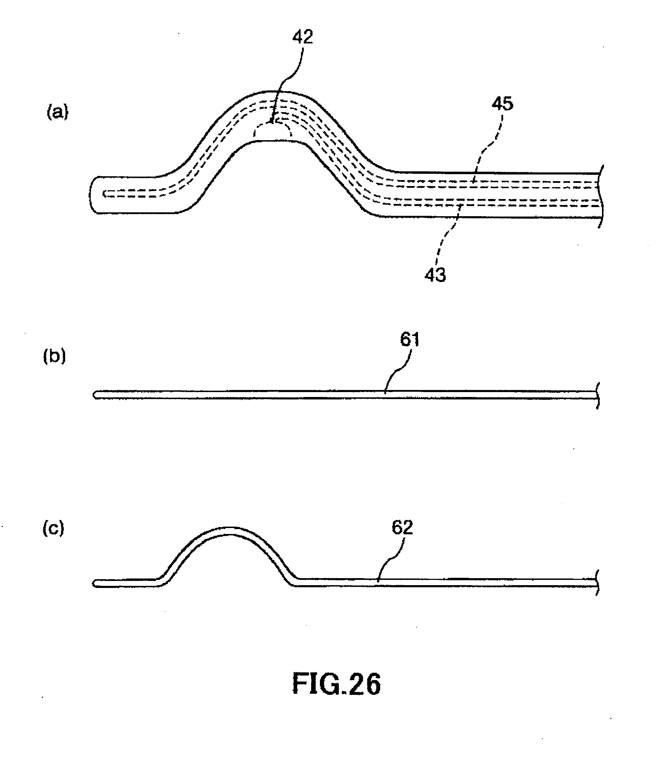

   FIG. 26 is a view depicting a urethral-insertion member and stylet in a seventh embodiment of the puncture apparatus of the present invention.

### Modes for Carrying Out the Invention

In the following, the puncture device and the puncture apparatus of the present invention is described in detail based on preferred embodiments depicted in the accompanying drawings.

### <First Embodiment>

FIG. 1 is a lateral view depicting a first embodiment of the puncture apparatus of the present invention. FIG. 2 is a cross sectional view taken along line A-A in FIG. 1. FIG. 3 is a cross sectional view of the puncture device of the puncture apparatus depicted in FIG. 1; FIG. 3(a) is a cross sectional view taken along line B-B in FIG. 1; FIG. 3(b) is a cross sectional view taken along line C-C in FIG. 3(a). FIG. 4 is a cross sectional view depicting a state in which a balloon catheter is inserted in a urethral-insertion member of the puncture apparatus depicted in FIG. 1. FIGS. 5 to 9 are views illustrating an operation procedure of the puncture apparatus depicted in FIG. 1.

Note that FIG. 5(a), FIG. 6(a), FIG. 7(a) and FIG. 8(a) are lateral views; FIG. 5(b) is a cross sectional view taken along line D-D in FIG. 5(a); FIG. 6(b) is a cross sectional view taken along line E-E in of FIG. 6(a); FIG. 7(b) is a cross sectional view taken along line F-F in FIG. 7(a); and FIG. 8(b) is a cross sectional view taken along line G-G in FIG. 8(a); and FIG. 9 is a view corresponding to the cross sectional view taken along line D-D in FIG. 5(a).

Further, in FIG. 5(b), FIG. 6(b), FIG. 7(b), FIG. 8(b) and FIG. 9, slanting lines for a living body are omitted so as to be easily viewable.

In the following, description is given assuming that the left side in FIG. 1, FIG. 4, FIG. 5(a), FIG. 6(a), FIG. 7(a) and FIG. 8(a) is "distal end" and the right side is "proximal end."

The puncture apparatus 1 depicted in the figures is an apparatus to be used for the treatment of woman's urinary incontinence, namely, to be used when a living body tissue supporting indwelling article for the treatment of urinary incontinence is implanted into the inside of the living body. The living body tissue supporting indwelling article is a implantable tool for the treatment of woman's urinary incontinence, namely, an elongated tool to be implanted into the living body for supporting the urethra (living body tissue), for example, a tool for supporting, for example, when it is intended to move the urethra to the vaginal wall side, the urethra tension-free or so as to pull the urethra in a direction in which the urethra is spaced away from the vaginal wall. For the living body tissue supporting indwelling article, it is possible to use, for example, an elongated object having flexibility.

As depicted in FIGS. 1 to 3, the puncture apparatus 1 includes a puncture device 10 for puncturing a living body tissue, a urethral-insertion member 4 of a longitudinal shape for being inserted into a urethra, a vaginal-insertion member 5 of a longitudinal shape for being inserted into a vagina, and a supporting member (restriction means) 2 for supporting the urethral-insertion member 4, the vaginal-insertion member 5 and a puncture member 3 (hereinafter described) of the puncture device 10.

The puncture device 10 includes a puncture needle 31 for puncturing a living body tissue, a puncture member 3 having a shaft portion 33 and a connection portion 32 for connecting the puncture needle 31 and the shaft portion 33 to each other, and an indwelling article assembly 8 having a needle tip portion 82 positioned at the distal end of the puncture needle 31 and configured to puncture a living body tissue and a living body tissue supporting indwelling article 81. The puncture needle 31 has a form of a pipe and has a hollow portion 312, and the living body tissue supporting indwelling article 81 is accommodated in the hollow portion 312.

The indwelling article assembly 8 has the living body tissue supporting indwelling article 81 in the form of a belt for attaching to a living body tissue, and the needle tip portion 82 fixed to a distal end portion of the living body tissue supporting indwelling article 81 for puncturing a living body tissue. The living body tissue supporting indwelling article 81 of the indwelling article assembly 8 is called "sling." In the present embodiment, the living body tissue supporting indwelling article 81 and the needle tip portion 82 are implanted into a living body.

The living body tissue supporting indwelling article 81 has a net-like form and can be configured, for example, as an article braided in a net-like form (lattice form) of crossing linear bodies, namely, as a braided body having a net-like form. The linear bodies may be, for example, those having a circular transverse sectional shape, those having a flattened transverse sectional shape, namely, those of a belt-like form (ribbon form), and so forth.

Note that it is needless to say that the living body tissue supporting indwelling article 81 is not limited to that of the net-like form described above.

The needle tip portion 82 has, at an apex portion thereof, a rounded and non-sharp needle tip for puncturing a living body tissue. Consequently, when the needle tip portion 82 is implanted in a living body, the living body tissue can be prevented from being damaged. The needle tip portion 82 is fixed at a proximal end portion thereof to the distal end portion of the living body tissue supporting indwelling article 81.

Further, a pair of protrusions 821 are formed at an outer circumferential portion of the proximal end portion of the needle tip portion 82 such that they protrude toward a direction of the proximal end. The protrusions 821 are arranged in an opposing relationship to each other across the center axis of the puncture needle 31. Further, the protrusions 821 are spaced from each other by a greater amount in the proximal end side than in the distal end side. Consequently, the needle tip portion 82 can be prevented from returning in the opposite direction to the puncturing direction of the needle tip portion 82, and an anchor effect can be obtained.

Note that the constituent materials of the needle tip portion 82 and the living body tissue supporting indwelling article 81 are not limited specifically and can be configured using, for example, various resin materials and so forth having biocompatibility.

In the indwelling article assembly 8, the living body tissue supporting indwelling article 81 is accommodated in the hollow portion 312 of the puncture needle 31, and the needle tip portion 82 is held removably, namely, detachably, at a distal end portion of the puncture needle 31.

In the present embodiment, the urethral-insertion member 4 is firmly fixed to the supporting member 2. The urethral-insertion member 4 has a straight tubular shape made of non-flexible hard material, and an opening at the proximal end thereof is open at the proximal end face of the supporting member 2. Into the urethral-insertion member 4, it is possible to insert various kinds of elongated medical tools such as, for example, a balloon catheter 11, which includes an expandable and contractible balloon 111 at the distal end portion thereof as depicted in FIG. 4. In FIG. 4, the balloon 111 in a contracted state is indicated by a solid line, and the balloon 111 in an expanded state is indicated by an alternate long and two-short dashes line.

The balloon 111 of the balloon catheter 11 functions as a restriction unit for restricting the position of the urethral-insertion member 4 in the axial direction (longitudinal direction) inside the urethra. More specifically, when the puncture apparatus 1 is to be used, the balloon 111 is inserted into the bladder of a patient, and the positional relation in the axial direction between the balloon catheter 11 and the urethral-insertion member 4 is fixed. Besides, the balloon 111 is caught by the bladder neck in a state in which the balloon 111 is expanded, and consequently, the position of the urethral-insertion member 4 with respect to the bladder and the urethra is fixed.

Note that a balloon expanding tool such as, for example, a syringe not depicted is connected to a port not depicted which communicates with a lumen not depicted which communicates with the balloon 111 of the balloon catheter 11. Then, operating fluid supplied from the balloon expanding tool is sent into or extracted from the inside of the balloon 111 thorough the aforementioned lumen to carry out expansion and contraction of the balloon 111. As the operating fluid for the expansion of the balloon, it is possible to use liquid such as, for example, physiological salt solution or gas.

Further, it is possible to use the balloon catheter 11 for the urination of the patient when the puncture apparatus 1 is to be used.

A marker 41 is provided at an outer circumferential portion of the urethral-insertion member 4. The marker 41 is arranged such that the marker 41 is positioned at the urethral orifice when the urethral-insertion member 4 is inserted into the urethra and the distal end portion of the urethral-insertion member 4 is positioned just in front of the bladder.

In the present embodiment, the vaginal-insertion member 5 is firmly fixed to the supporting member 2. Note that the vaginal-insertion member 5 may otherwise be removably provided on the supporting member 2. The vaginal-insertion member 5 has a form of a straight bar. Further, the distal end portion of the vaginal-insertion member 5 is rounded. Consequently, it is possible to insert the vaginal-insertion member 5 smoothly into the vagina.

Further, the vaginal-insertion member 5 is arranged in a spaced relationship by a predetermined distance from the urethral-insertion member 4 below the urethral-insertion member 4 such that the axial line thereof and the axial line of the urethral-insertion member 4 extend in parallel to each other.

Note that the constituent materials of the vaginal-insertion member 5, the urethral-insertion member 4 and the supporting member 2 are not limited specifically, and it is possible to use, for example, various kinds of resin materials.

The puncture member 3 of the puncture device 10 is installed, at the shaft portion 33 thereof which serves as a rotational shaft, for rotational movement on the supporting member 2.

Further, the shaft portion 33 is arranged in a spaced relationship by a predetermined distance from the urethral-insertion member 4 above the urethral-insertion member 4 such that the axial line thereof and the axial line of the urethral-insertion member 4 extend in parallel to each other. Further, as viewed from an axial direction of the shaft portion 33, the shaft portion 33, urethral-insertion member 4 and vaginal insertion member 5 are arranged on a straight line.

The shaft portion 33 passes through the supporting member 2 in the leftward and rightward direction in FIG. 1. On the distal end and the proximal end of the shaft portion 33, a flange 331 and another flange 332 are formed, respectively, with the supporting member 2 interposed therebetween. The movement of the shaft portion 33 in the axial direction with respect to the supporting member 2 is blocked by the flanges 331 and 332.

The puncture needle 31 has a form of a pipe and has a hollow portion 312. The hollow portion 312 is open at the distal end and closed at the proximal end thereof. Further, the puncture needle 31 has a flattened shape as viewed in the longitudinal direction thereof. In other words, the transverse sectional shape of the puncture needle 31 corresponds to the transverse sectional shape of the living body tissue supporting indwelling article 81. Consequently, a puncture hole 500 of a shape corresponding to that of the living body tissue supporting indwelling article 81 can be formed in a patient, and the indwelling article assembly 8 can be implanted more readily and appropriately.

Further, the puncture needle 31 is curved in an arc centered at the shaft portion 33. Further, in FIG. 1, the axial line of the puncture needle 31 and the axial line of the shaft portion 33 cross orthogonally with each other. Consequently, when the puncture member 3 is rotationally moved, the needle tip of the puncture needle 31 moves along the arc described above in a plane perpendicular to the axial line of the shaft portion 33, namely, in a plane having a normal line at the axial line.

Further, while, in the present embodiment, the distal end of the puncture needle 31 is directed in a counterclockwise direction in FIG. 2, the direction of the distal end of the puncture needle 31 is not limited to this and the distal end of the puncture needle 31 may be directed in a clockwise direction in FIG. 2.

Further, in the present embodiment, the puncture needle 31 is arranged at the proximal end side with respect to a distal end portion of the urethral-insertion member 4 in the axial direction of the urethral-insertion member 4.

Note that the puncture needle 31 may be arranged at a position same as that of a distal end portion of the urethral-insertion member 4 in the axial direction of the urethral-insertion member 4 or may be arranged at the distal end side with respect to the distal end portion of the urethral-insertion member 4.

Here, the supporting member 2 restricts the positional relationship between the puncture device 10 and the urethral-insertion member 4 so that, when the puncture device 10 is rotationally moved and punctuates a living body tissue, the needle tip of the needle tip portion 82 passes at the farther-position side from the center 311 of the puncture needle 31 than the urethral-insertion member 4 or an extension line of the urethral-insertion member 4, namely, below the urethral-insertion member 4 or the extension line of the urethral-insertion member 4. Note that the center 311 of the puncture needle 31 is the center of the arc of the puncture needle 31. In other words, the center 311 of the puncture needle 31 is the center of the rotational movement of the puncture needle 31 (puncture device 10).

Further, the supporting member 2 restricts the positional relationship between the puncture device 10 and the vaginal insertion member 5 so that, when the puncture device 10 is rotationally moved and punctures a living body, the needle tip of the needle tip portion 82 may not collide with the vaginal insertion member 5 and an extension line of the vaginal insertion member 5.

In particular, the supporting member 2 restricts the positional relationship among the puncture device 10, urethral-insertion member 4 and vaginal insertion member 5 so that, when the puncture device 10 is rotationally moved and punctures a living body, the needle tip of the needle tip portion 82 passes between the urethral-insertion member 4 or the extension line of the urethral-insertion member 4 and the vaginal insertion member 5 or the extension line of the vaginal insertion member 5.

Consequently, the puncture device 10 can puncture a living body tissue avoiding the urethra and the vaginal wall, and the puncture device 10 can be prevented from puncturing the urethra and from puncturing the vaginal wall.

Further, since the trajectory of the needle tip of the needle tip portion 82 of the puncture device 10 is determined, also the operator can safely prevent the operator itself from being punctured at the fingertip thereof by the puncture device 10.

Further, although the center angle of the arc of the puncture needle 31 is not restricted particularly but is set suitably in response to various conditions, the center angle is set such that, when a living body tissue is punctured by the puncture device 10, the needle tip portion 82 of the puncture device 10 enters into the body from the body surface at one side of a patient, passes below the urethra and can move to a position in the proximity of the body surface at another side of the patient.

In particular, the center angle θ1 of the arc of the puncture needle 31 and the needle tip portion 82 preferably is 150 to 270 degrees, more preferably is 170 to 250 degrees, and most preferably is 190 to 230 degrees.

Consequently, when a living body tissue is punctured by the puncture device 10, the needle tip portion 82 can enter into the body from the body surface at one side of the patient, pass below the urethra and be positioned in the proximity of the body surface at the other side without penetrating the body surface at the other side.

Further, a grip unit 34 is provided as an operation unit for operating the puncture device 10 to move rotationally is provided at a proximal end portion of the shaft portion 33. The shape of the grip unit 34 in the present embodiment has a form of a rectangular solid. When the puncture device 10 is to be moved rotationally, the grip unit 34 is gripped by the fingers of a hand and is moved rotationally in a predetermined direction. Note that it is needless to say that the shape of the grip unit 34 is not to be limited to this.

Note that the constituent material of the puncture member 3 is not limited specifically, and such various metal materials as stainless steel, aluminum or aluminum alloy and titanium or titanium alloy can be used as the constituent material of the puncture member 3.

Now, an operation procedure of the puncture apparatus 1, namely, a procedure when the indwelling article assembly 8 is implanted into a living body, is described.

First, the puncture apparatus 1 is mounted on a patient as depicted in FIG. 5. In particular, the urethral-insertion member 4 of the puncture apparatus 1 is inserted into the urethra 100 of the patient and the vaginal-insertion member 5 is inserted into the vagina 200 of the patient. At the time, the insertion is carried out such that the marker 41 is positioned at the urethral orifice or on the front side of the urethral orifice. Consequently, the distal end portion of the urethral-insertion member 4 can be arranged on the front side of the bladder.

Then, the grip unit 34 is grasped as depicted in FIGS. 6 and 7 and rotationally moves the puncture device 10 counterclockwise in FIG. 6(b) and FIG. 7(b).

Consequently, the needle tip of the needle tip portion 82 of the puncture device 10 moves counterclockwise in FIG. 6(b) and FIG. 7(b) along the arc thereof; punctures the body surface at an inguinal region of the patient on the left side in FIG. 6(b) and FIG. 7(b) or at a region in the vicinity of the same; enters into the body; passes an obturator foramen 400a of a pelvis 300; passes below the urethra 100, namely, passes between the urethra 100 and the vagina 200; passes an obturator foramen 400b of the pelvis 300; and moves to the proximity of an inguinal region on the right side in FIG. 6(b) and FIG. 7(b) or at a region in the proximity of the inguinal region. Note that the needle tip of the needle tip portion 82 does not penetrate the body surface at the inguinal region on the right side or at a region in the proximity of the inguinal region. Consequently, in the patient, a puncture hole 500 is formed which extends from the body surface at the inguinal region at the left side in FIG. 6(b) and FIG. 7(b) or at a region in the proximity of the inguinal region past the obturator foramen 400a, a region between the urethra 100 and the vagina 200 and the obturator foramen 400b to a position in the proximity of the body surface at the inguinal region at the right side in FIG. 6(b) and FIG. 7(b) or at a region in the proximity of the inguinal region.

Then, as depicted in FIG. 8, the grip unit 34 is grasped and the puncture device 10 is rotationally moved clockwise in FIG. 8.

Thereupon, the indwelling article assembly 8 is prevented from returning in the opposite direction to the puncturing direction by the protrusions 821 of the needle tip portion 82 thereof. Further, the distal end of the puncture needle 31 moves in the clockwise direction in FIG. 8 along the arc thereof; passes through the obturator foramen 400b of the pelvis 300; passes below the urethra 100; namely; passes a location between the urethra 100 and the vagina 200; passes through the obturator foramen 400a of the pelvis 300; and comes out to the outside of the body from the body surface at the inguinal region at the left side in FIG. 8 or at a region in the proximity of the inguinal region. In other words, the puncture needle 31 is pulled out to the outside of the body. The indwelling article assembly 8, namely, the living body tissue supporting indwelling article 81, is implanted into the living body in such a manner as described above.

Then, the puncture apparatus 1 is removed from the patient. In particular, the urethral-insertion member 4 is pulled out from within the urethra 100 and the vaginal insertion member 5 is pulled out from within the vagina 200 of the patient. Then, predetermined treatment is carried out, thereby ending the manual procedure.

As described above, with the puncture apparatus 1, when the living body tissue supporting indwelling article 81 is to be indwelled, the indwelling operation can be carried out only by a low invasive manual procedure such as puncturing of the puncture device 10. Since it is not necessary to carry out a high invasive incision or the like, the burden on the patient is light and also the safety of the patient is high.

Further, it is possible to puncture the living body by the puncture device 10 avoiding the urethra and the vaginal wall, and it is possible to prevent the puncture device 10 from puncturing the urethra and puncturing the vaginal wall. Therefore, safety can be achieved. Also the operator itself can prevent its fingertip from being punctured by the puncture device 10. Therefore, safety can be achieved.

Further, it is possible to prevent such a situation as in a conventional case in which the vagina is incised that the living body tissue supporting indwelling article 81 is exposed to the inside of the vagina through a wound caused by the incision or that such complications as an infection from the wound occur. Therefore, the living body tissue supporting indwelling article 81 can be implanted in very high safety and with certainty.

Further, since the living body tissue supporting indwelling article 81 is accommodated in the hollow portion 312 of the puncture needle 31, the living body tissue supporting indwelling article 81 can be implanted readily.

Note that, although, in the present embodiment, one end portion of the puncture hole formed in the patient by the puncture device does not penetrate the body surface, the puncture hole is not limited to this and may be a through-hole.

Further, the urethral-insertion member is not limited to that of a tubular shape, and may be, for example, a solid member or may be a hollow member which is closed up at one or both of the distal end portion and the proximal end portion thereof.

Further, at the distal end portion of the urethral-insertion member, an expandable and contractible balloon may be provided as a restriction unit for restricting the position of the urethral-insertion member in the axial direction inside the urethra.

Further, while the puncture needle of the puncture device in the present embodiment is curved in an arc over an overall extent thereof, the puncture needle is not limited to this and may have an arcuately curved portion only at part thereof. In other words, only it is necessary for the puncture needle to have an arcuately curved portion at least at part thereof.

Further, the puncture needle may have a curved portion at least at part thereof, and, for example, may be curved in an elliptical arc over an overall extent thereof or may have a portion curved in an elliptical arc only at part thereof. In other words, the puncture needle may have a portion curved in an elliptical arc at least at part thereof.

### <Second Embodiment>

FIG. 10 is a side elevational view depicting a second embodiment of the puncture apparatus of the present invention. FIG. 11 is a cross sectional view taken along line H-H in FIG. 10. FIG. 12 is a cross sectional view of a puncture device of the puncture apparatus. FIGS. 13 to 19 are views illustrating an operation procedure of the puncture apparatus.

Note that FIG. 13(a), FIG. 14(a) and FIG. 15(a) are lateral views; FIG. 13(b) is a cross sectional view taken along line I-I in FIG. 13(a); FIG. 14(b) is a cross sectional view taken along line J-J in FIG. 14(a); FIG. 15(b) is a cross sectional view taken along line K-K in FIG. 15(a); and FIGS. 16 to 19 correspond to the cross sectional view taken along line I-I in FIG. 13(a).

Further, in FIG. 13(b), FIG. 14(b), FIG. 15(b) and FIGS. 16 to 19, slanting lines for a living body are omitted so as to be easily viewable.

The following description is given assuming that the left side in FIG. 10, FIG. 13(a), FIG. 14(a), and FIG. 15(a) is "distal end" and the right side is "proximal end."

In the following, the second embodiment is described principally in regard to differences thereof from the first embodiment described hereinabove while description of similar matters is omitted.

As depicted in FIGS. 10 to 12, in the puncture apparatus 1 of the second embodiment, the living body tissue supporting indwelling article 81a of the indwelling article assembly 8 includes an indwelling article main body 810, a string (first string member) 83 connected at the proximal end thereof to the distal end of the indwelling article main body 810, a string (second string member) 84 connected at the distal end thereof to the proximal end of the indwelling article main body 810, and a holding member 85 connected to the proximal end of the string 84. In particular, the string 83 is fixed at a proximal end portion thereof to a distal end portion of the indwelling article main body 810, and the string 84 is fixed at a distal end portion thereof to a proximal end portion of the indwelling article main body 810. Further, the holding member 85 is fixed to a proximal end portion of the string 84 and is held removably, namely, detachably, on the hollow portion 312 at a proximal end portion of the puncture needle 31. Note that the indwelling article main body 810 is similar to the living body tissue supporting indwelling article 81 in the first embodiment.

The holding member 85 in the present embodiment has a spherical shape. Note that it is needless to say that the shape of the holding member 85 is not limited to this.

Further, the constituent materials of the strings 83 and 84 are not limited specifically and can be configured, for example, using various resin materials, fiber materials and so forth. In addition, the constituent material of the holding member 85 is not limited specifically and can be configured, for example, using various resin materials.

Further, a needle tip portion 86 of the indwelling article assembly 8 has a sharp needle tip at the distal end thereof. This needle tip portion 86 is fixed to a distal end portion of the string 83.

In the present embodiment, the needle tip portion 86 and the holding member 85 of the indwelling article assembly 8 are not implanted in a living body, but the indwelling article main body 810 of the living body tissue supporting indwelling article 81a and part of the strings 83 and 84 are implanted in a living body.

Further, the center angle of the arc of the puncture needle 31 is not limited specifically and is set suitably in response to various conditions. However, the center angle is set such that, when the puncture device 10 punctures a living body tissue, the needle tip portion 86 of the puncture device 10 can enter into the body from the body surface at one side of the patient, pass below the urethra and protrude to the outside of the body from the body surface at the other side.

In particular, the center angle θ2 of the arc of the puncture needle 31 and the needle tip portion 86 preferably is 150 to 270 degrees, more preferably is 170 to 250 degrees, and most preferably is 190 to 230 degrees.

Consequently, when the puncture device 10 punctures a living body tissue, the needle tip portion 86 can enter into the body from the body surface at one side of the patient, pass below the urethra and protrude to the outside of the body from the body surface at the other side with certainty.

Now, an operation procedure of the puncture apparatus 1, namely, a procedure when the indwelling article main body 810 of the indwelling article assembly 8 is implanted into a living body, is described.

First, the puncture apparatus 1 is mounted on a patient as depicted in FIG. 13. In particular, the urethral-insertion member 4 of the puncture apparatus 1 is inserted into the urethra 100 of the patient and the vaginal-insertion member 5 is inserted into the vagina 200 of the patient. At the time, the insertion is carried out such that the marker 41 is positioned at the urethral orifice or on the front side of the urethral orifice. Consequently, the distal end portion of the urethral-insertion member 4 can be arranged on the front side of the bladder.

Then, the grip unit 34 is grasped as depicted in FIGS. 14 and 15 and rotationally moves the puncture device 10 counterclockwise in FIG. 14(b) and FIG. 15(b).

Consequently, the needle tip of the needle tip portion 86 of the puncture device 10 moves counterclockwise in FIG. 14(b) and FIG. 15(b) along the arc thereof; punctures the body surface at an inguinal region of the patient on the left side in FIG. 15(b) and FIG. 16(b) or at a region in the vicinity of the same; enters into the body; passes an obturator foramen 400a of a pelvis 300; passes below the urethra 100, namely, passes between the urethra 100 and the vagina 200; passes an obturator foramen 400b of the pelvis 300; and protrudes to the outside of the body from the body surface at the inguinal region at the right side in FIG. 14(b) and FIG. 15(b) or at a region in the proximity of the inguinal region. Consequently, in the patient, a puncture hole 600 is formed which extends from the body surface at an inguinal region on the left side in FIG. 14(b) and FIG. 15(b) or at a region in the vicinity of the same to the body surface at an inguinal region on the right side in FIG. 14(b) and FIG. 15(b) or at a region in the vicinity of the same while passing through the obturator foramen 400a, between the urethra 100 and the vagina 200 and through the obturator foramen 400b.

Then, the needle tip portion 86 is removed from the distal end portion of the puncture needle 31 as depicted in FIG. 16, and the grip unit 34 is grasped as depicted in FIG. 17 to rotationally move the puncture device 10 clockwise in FIG. 8.

Consequently, the distal end of the puncture needle 31 moves clockwise in FIG. 17 along the arc thereof; passes through the obturator foramen 400b of the pelvis 300; passes below the urethra 100, namely, passes between the urethra 100 and the vagina 200; passes through the obturator foramen 400a of the pelvis 300; and goes out to the outside of the body from the body surface at an inguinal region at the left side in FIG. 17 or at a region in the proximity of the same. That is, the puncture needle 31 is removed to the outside of the body.

Then, the holding member 85 is removed from the distal end of the hollow portion 312 of the puncture needle 31. Further, the puncture apparatus 1 is removed from the patient. In particular, the urethral-insertion member 4 is pulled out from within the urethra 100, and the vaginal insertion member 5 is pulled out from within the vagina 200 of the patient.

Then, the strings 83 and 84 are pulled individually by predetermined forces as depicted in FIG. 18 to adjust the position of the indwelling article main body 810 with respect to the urethra 100, and unnecessary portions of the strings 83 and 84 are cut away. Then, predetermined treatment is carried out, thereby ending the manual procedure. The indwelling article main body 810 is implanted into the living body tissue in this manner.

With the present puncture apparatus 1, similar effects to those by the first embodiment described hereinabove are achieved.

Note that, while the needle tip portion in the present embodiment is fixed to a distal end portion of a string, the fixation of the needle tip portion is not limited to this, and the needle tip portion may be held removably, namely, detachably, on a string.

### <Third Embodiment>

FIG. 20 is a cross sectional view depicting a puncture device according to a third embodiment of the puncture apparatus of the present invention.

In the following, the third embodiment is described principally in regard to differences thereof from the second embodiment described hereinabove while description of similar items is omitted.

As depicted in FIG. 20, in the puncture apparatus 1 of the third embodiment, the puncture needle 31 of the puncture device 10 has a needle tip portion 35 at a distal end portion thereof. In other words, the needle tip portion 35 is fixed to or integrated with the distal end portion of the puncture needle 31. Further, the needle tip portion 35 has a sharp needle tip at the distal end thereof. Further, the hollow portion 312 of the puncture needle 31 is open at both of the distal end and the proximal end thereof. Further, the puncture device 10 has a pusher not depicted for pushing the string 83 of the living body tissue supporting indwelling article 81a in a direction toward the distal end.

In the present puncture apparatus 1, when it is to be used, the pusher is inserted into the hollow portion 312 from the proximal end opening of the puncture needle 31, and the string 83 of the living body tissue supporting indwelling article 81a is pushed to move in the direction toward the distal end by the pusher so that the string 83 protrudes from the distal end opening 351 of a needle tip portion 35.

With the present puncture apparatus 1, similar effects to those by the second embodiment described hereinabove are achieved.

### <Fourth Embodiment>

FIG. 21 is a cross sectional view depicting a puncture device according to a fourth embodiment of the puncture apparatus of the present invention.

In the following, the fourth embodiment is described principally in regard to differences thereof from the first embodiment described hereinabove while description of similar items is omitted.

In the puncture apparatus 1 of the fourth embodiment depicted in FIG. 21, the puncture needle 31 has a variable length. In particular, the puncture needle 31 includes a needle main body 313 for puncturing a living body tissue, and an extension needle 314 provided for relative movement to the needle main body 313 along a longitudinal direction of the needle main body 313 for puncturing a living body tissue. In other words, the puncture needle 31 is extended by the extension needle 314 moving in a direction toward the distal end of the needle main body 313 with respect to the needle main body 313. Further, a hollow portion of the needle main body 313 and the extension needle 314 is open at the distal end and the proximal end thereof.

Further, the needle tip portion 82 of the indwelling article assembly 8 is held removably, namely, detachably, at a distal end portion of the extension needle 314.

Further, the puncture device 10 includes, as extension means for moving the extension needle 314 in the direction toward the distal end of the needle main body 313 with respect to the needle main body 313, a pusher not depicted which pushes the extension needle 314 to move to the direction toward the distal end of the needle main body 313.

In the present puncture apparatus 1, when it is to be used, the pusher is inserted into the hollow portion of the extension needle 314 from the proximal end opening so that the extension needle 314 is pushed in the direction toward the distal end thereof by the pusher to extend the puncture needle 31.

With the present puncture apparatus 1, similar effects to those by the first embodiment described hereinabove are achieved.

### <Fifth Embodiment>

FIG. 22 is a cross sectional view depicting a puncture device in a fifth embodiment of the puncture apparatus of the present invention.

In the following, the fifth embodiment is described principally in regard to differences thereof from the second embodiment described hereinabove while description of similar items is omitted.

In the puncture apparatus 1 of the fifth embodiment depicted in FIG. 22, the puncture needle 31 has a variable length. In particular, the puncture needle 31 includes a needle main body 313 for puncturing a living body tissue, and an extension needle 315 provided for relative movement to the needle main body 313 along a longitudinal direction of the needle main body 313 for puncturing a living body tissue. In particular, the puncture needle 31 is extended by movement of the extension needle 315 in a direction toward the distal end of the needle main body 313 with respect to the needle main body 313. Further, a hollow portion of the needle main body 313 and the extension needle 315 is open at both of the distal end and the proximal end thereof.

Further, the extension needle 315 has a needle tip portion 35 at a distal end portion thereof. In other words, the needle tip portion 35 is fixed to or integrated with the distal end portion of the extension needle 315. Further, the needle tip portion 35 has a sharp needle tip at the distal end thereof. Further, the puncture device 10 has a first pusher not depicted for pushing the string 83 of the living body tissue supporting indwelling article 81a to move in a direction toward the distal end.

In the present puncture apparatus 1, when it is used, the first pusher is inserted into the hollow portion of the extension needle 315 from the proximal end opening so that the string 83 of the living body tissue supporting indwelling article 81a is pushed in the direction toward the distal end by the first pusher to protrude from the distal end opening 351 of the needle tip portion 35.

Further, the puncture device 10 includes, as extension means for moving the extension needle 315 in the direction toward the distal end of the needle main body 313 with respect to the needle main body 313 to extend the puncture needle 31, a second pusher for pushing the extension needle 315 to move in the direction toward the distal end of the needle main body 313.

In the present puncture apparatus 1, when it is to be used, the second pusher is inserted into the hollow portion of the extension needle 315 from the proximal end opening of the same so that the extension needle 315 is pushed in the direction toward the distal end by the second pusher to extend the puncture needle 31.

With the present puncture apparatus 1, similar effects to those by the second embodiment described hereinabove are achieved.

### <Sixth Embodiment>

FIG. 23 is a side elevational view depicting a sixth embodiment of the puncture apparatus of the present invention. FIG. 24 is a bottom plan view depicting a urethral insertion member of the puncture apparatus depicted in FIG. 23, namely, a view of the urethral-insertion member as viewed from the lower side in FIG. 23. FIG. 25 is a plan view depicting a vaginal insertion member of the puncture apparatus depicted in FIG. 23, namely, a view of the vaginal insertion member as viewed from the upper side in FIG. 23.

Note that, in the following, description is given assuming that the left side in FIG. 23 is "distal end"; the right side is "proximal end"; the upper side is "upper"; and the lower side is "lower."

In the following, the sixth embodiment is described principally in regard to differences thereof from the first embodiment described hereinabove while description of similar items is omitted.

As depicted in FIG. 23, the puncture apparatus 1 of the sixth embodiment is configured such that the positional relationship between the urethral-insertion member 4 and the vaginal insertion member 5 is changeable, in other words, the angle of the axial line of the urethral-insertion member 4 is changeable. In particular, the supporting member 2 includes a urethral-insertion member supporting mechanism 21 capable of supporting the urethral-insertion member 4 with the angle of the axial line of the urethral-insertion member 4 changed. The urethral-insertion member supporting mechanism 21 is configured such that it can support the urethral-insertion member 4 in such a manner that a distal end portion of the urethral-insertion member 4 is directed obliquely upwardly as indicated by an alternate long and two short dashes line in FIG. 23 and can support the urethral-insertion member 4 in such a manner that the distal end portion of the urethral-insertion member 4 is directed obliquely downwardly.

Further, in the puncture apparatus 1, the angle of the axial line of the vaginal insertion member 5 can be changed. In particular, the supporting member 2 includes a vaginal insertion member supporting mechanism 22 which can support the vaginal insertion member 5 with the angle of the axial line of the vaginal insertion member 5 changed. The vaginal insertion member supporting mechanism 22 is configured such that it can support the vaginal insertion member 5 in such a manner that a distal end portion of the vaginal insertion member 5 is directed obliquely downwardly as indicated by an alternate long and two short dashes line in FIG. 23 and can support the vaginal insertion member 5 in such a manner that the distal end portion of the vaginal insertion member 5 is directed obliquely upwardly.

Consequently, as indicated by the alternate long and two short dashes line in FIG. 23, the thickness of a living body tissue between the urethra and the vagina can be increased by arranging the urethral-insertion member 4 and the vaginal insertion member 5 so as to have inclined axial lines such that the distance between the axial line of the urethral-insertion member 4 and the axial line of the vaginal insertion member 5 increases toward the distal end side. Consequently, the puncture device 10 can puncture a living body tissue between the urethra and the vagina readily and with certainty.

Further, though not depicted, also it is possible to arrange the urethral-insertion member 4 and the vaginal insertion member 5 so as to have inclined axial lines such that the distance between the axial line of the urethral-insertion member 4 and the axial line of the vaginal insertion member 5 decreases toward the distal end side.

Note that the urethral-insertion member supporting mechanism 21 and the vaginal insertion member supporting mechanism 22 configure positional relationship changing means for changing the positional relationship between the urethral-insertion member 4 and the vaginal insertion member 5.

Meanwhile, the urethral-insertion member 4 has a recessed portion 42 at a lower face of a distal end portion thereof.

Further, the urethral-insertion member 4 has a lumen 43 formed along an axial direction thereof. The lumen 43 is open to the proximal end of the urethral-insertion member 4 and to a bottom face of the recessed portion 42, namely, to an upper face. Further, a port 44 is provided at the proximal end of the urethral-insertion member 4 such that it is communicated with the lumen 43. A pump not depicted is connected to the port 44 through a pipe member not depicted. By carrying out suction by operation of the pump, a living body tissue in the urethra, namely, the inner wall of the urethra, can be attracted. Consequently, the inner wall of the urethra can be pulled with certainty by the urethral-insertion member 4, and the thickness of a living body tissue between the urethra and the vagina can be increased. Note that the recessed portion 42, lumen 43, port 44, housing and pump configure suction means.

Further, the vaginal insertion member 5 has a recessed portion 51 at an upper face of a distal end portion thereof.

Further, the vaginal insertion member 5 has a lumen 52 formed along an axial direction thereof. The lumen 52 is open to the proximal end of the vaginal insertion member 5 and a bottom face of the recessed portion 51, namely, a lower face of the recessed portion 51. A port 53 is provided at the proximal end of the vaginal insertion member 5 such that it is communicated with the lumen 52. A pump not depicted is connected to the port 53 through a housing not depicted such that, by carrying out suction by operation of the pump, a living body tissue in the vagina in the recessed portion 51, namely, the inner wall of the vagina, can be attracted. Consequently, the inner wall of the vagina can be pulled with certainty by the vaginal insertion member 5, and the thickness of the living body tissue between the urethra and the vagina can be increased. Note that suction means is configured from the recessed portion 51, lumen 52, port 53, housing and pump.

With the present puncture apparatus 1, effects similar to those by the first embodiment can be achieved.

### <Seventh Embodiment>

FIG. 26 is a view depicting a urethral-insertion member and a stylet in a seventh embodiment of the puncture apparatus of the present invention. FIG. 26(a) is a cross sectional view depicting the urethra-insertion member, and FIGS. 26(b) and 26(c) are side elevational views depicting the stylet.

Note that, in the following, description is given assuming that the left side in FIG. 26 is "distal end"; the right side is "proximal end"; the upper side is "upper"; and the lower side is "lower."

In the following, the seventh embodiment is described principally in regard to differences thereof from the sixth embodiment described hereinabove while description of similar items is omitted.

As depicted in FIG. 26, the puncture apparatus 1 of the seventh embodiment includes a linear stylet 61 having a linear shape, and a curved stylet 62 curved at a distal end portion thereof.

Further, the urethral-insertion member 4 is configured from a tube member having flexibility and exhibiting a substantially linear state in a natural state in which no external force is applied thereto. The urethral-insertion member 4 includes, in addition to the lumen 43, a lumen 45 into which the linear stylet 61 and the curved stylet 62 are selectively inserted. The lumen 45 is formed to extend from the proximal end to a distal end portion of the urethral-insertion member 4. Further, the lumen 45 is closed up at the distal end thereof but is open at the proximal end thereof to the proximal end of the urethral-insertion member 4.

A port not depicted is provided at the proximal end of the urethral-insertion member 4 such that it is communicated with the lumen 45 so that the linear stylet 61 or the curved stylet 62 can be inserted from the port.

If the linear stylet 61 is inserted into the lumen 45, then the urethral-insertion member 4 is placed into a linear state by the linear stylet 61. Consequently, the urethral-insertion member 4 can be inserted into the urethra readily and smoothly.

On the other hand, if the curved stylet 62 is inserted into the lumen 45, then the distal end portion of the urethral-insertion member 4 is curved by the curved stylet 62. Consequently, the thickness of a living body tissue between the urethra and the vagina can be increased. Consequently, the puncture device 10 can puncture a living body tissue between the urethra and the vagina readily and with certainty.

With the present puncture apparatus 1, similar to effects to those by the sixth embodiment described hereinabove can be achieved.

Although the puncture device and the puncture apparatus of the present invention have been described on the basis of the embodiments depicted in the drawings, the present invention is not limited to them, and the configuration of each unit can be replaced with an arbitrary configuration having a similar function. Further, other arbitrary constructions may be added to the present invention.

Further, the present invention may be a combination of arbitrary two or more configurations of the embodiments described above.

Further, in the present invention, for example, the vaginal insertion member may be omitted while the restriction means is configured such that it restricts only the positional relationship between the puncture needle (puncture member) and the vaginal insertion member.

Note that, in the description of the embodiments described above, the puncture device and the puncture apparatus of the present invention are applied to an apparatus which is used when a living body tissue supporting indwelling article which can be implanted for the treatment of the woman's urinary incontinence is implanted into the living body. However, the application of the puncture device and the puncture apparatus of the present invention is not limited to this.

For example, the target of the application of the present invention includes an excretory disorder along with the weakening of the pelvic floor muscle group (urinary urgency, frequent urination, urinary incontinence, fecal incontinence, urinary retention, dysuria or the like), and a pelvic floor disorder including pelvic organ prolapse, vesicovaginal fistula, urethrovaginal fistula, pelvic pain or the like. In the pelvic organ prolapse, there are include disorders of cystocele, enterocele, rectocele, hysterocele and the like. Alternatively, there are included disorders of anterior vaginal prolapse, posterior vaginal prolapse, vaginal vault prolapse, vaginal apical prolapse and the like in which the naming method thereof is based on the manipulating vaginal-wall regions.

Also, in the overactive tissues, there are included bladder, vagina, uterus, bowel and the like. In the lessactive tissues, there are included bones, muscles, fascias, ligaments and the like. In particularly, in the pelvic floor disorders, there are included an obturator fascia, a coccygeus fascia, a cardinal ligament, an uterosacral ligament, a sacrotuberous ligament and the like.

For the procedure for interlocking an overactive tissue in the pelvic floor disorder with the lessactive tissue, there are included a retropubic sling surgery, a transobturator sling surgery (transobturator sling surgery, transobturator tape: TOT), a tension-free vaginal mesh (Tension-free Vaginal Mesh: TVM) surgery, a uterosacral ligament suspension (Uterosacral Ligament Suspension: USLS) surgery, a sacrospinous ligament fixation (Sacrospinous Ligament Fixation: SSLF) surgery, an iliococcygeus fascia fixation surgery, a coccygeus fascia fixation surgery, and the like.

### Industrial Applicability

The puncture device of the present invention includes:
a puncture needle having a hollow portion;
a needle tip portion positioned at a distal end of the puncture needle and configured to puncture a living body tissue; and
an elongated living body tissue supporting indwelling article accommodated in the hollow portion of the puncture needle and configured to be implanted into a living body to support the living body tissue.

Meanwhile, the puncture apparatus of the present invention includes:
the puncture device according to the present invention;
a urethral-insertion member of a longitudinal shape configured to be inserted into a urethra; and
restriction means for restricting, when the puncture device is rotationally moved to puncture a living body tissue, a positional relationship between the puncture device and the urethral-insertion member such that the needle tip portion passes at a farther-position side from the center of rotational movement of the puncture device than the urethral-insertion member.

With the present invention, it is possible to implant a living body tissue supporting indwelling article into a living body readily, and when the living body tissue supporting indwelling article is implanted, the burden on the patient is light and the safety of the patient is high. Also the safety of the operator is high.

Especially, where the puncture apparatus includes the restriction means for restricting the positional relation between the puncture device and the urethral-insertion member such that, when the puncture device rotationally moves to puncture the living body tissue, the needle tip portion passes the farther-position side from the center of rotational movement of the puncture device than the urethral-insertion member, for example, when the puncture apparatus is to be used for the treatment of woman's urinary incontinence, the urethral-insertion member of the puncture apparatus is inserted into a urethra, and the puncture device is rotationally moved so that the living body is punctured by the puncture device. Thereupon, since the needle tip portion passes the farther-position side from the center of the puncture device than the urethral-insertion member, the puncture device can puncture the living body avoiding the urethra. Consequently, the puncture device can be prevented from puncturing the urethra. Further, it is possible to prevent a fingertip of the operator from being punctured by the puncture device.

Further, when the living body tissue supporting indwelling article for the treatment of urinary incontinence is to be implanted, no incision of the vaginal wall is required, and the living body tissue supporting indwelling article can be implanted by a low invasive manual procedure. Further, such a situation that, as in a case in which the vagina is incised, the living body tissue supporting indwelling article is exposed to the inside of the vagina through a wound caused by the incision or that such complications as an infection from the wound occur can be prevented. Therefore, the living body tissue supporting indwelling article can be implanted in very high safety and with certainty.

Further, since the living body tissue supporting indwelling article is accommodated in the hollow portion of the puncture needle, the living body tissue supporting indwelling article can be implanted into a living body readily.

Accordingly, the present invention has industrial applicability.

### Description of Reference Numerals

- 1:: Puncture apparatus
- 2:: Supporting member
- 21:: Urethral-insertion member supporting mechanism
- 22:: Vaginal insertion member supporting mechanism
- 3:: Puncture member
- 31:: Puncture needle
- 311:: Center
- 312:: Hollow portion
- 313:: Main body
- 314, 315:: Extension needle
- 32:: Connection portion
- 33:: Shaft portion
- 331, 332:: Flange
- 34:: Grip unit
- 35:: Needle tip portion
- 351:: Distal end opening
- 4:: Urethral-insertion member
- 41:: Marker
- 42:: Recessed portion
- 43, 45:: Lumen
- 44:: Port
- 5:: Vaginal insertion member
- 51:: Recessed portion
- 52:: Lumen
- 53:: Port
- 61:: Linear stylet
- 62:: Curved stylet
- 8:: Indwelling article assembly
- 81, 81a:: Living body tissue supporting indwelling article
- 810:: Indwelling article main body
- 82:: Needle tip portion
- 821:: Protrusion
- 83, 84:: String
- 85:: Holding member
- 86:: Needle tip portion
- 10:: Puncture device
- 11:: Balloon catheter
- 111:: Balloon
- 100:: Urethra
- 200:: Vagina
- 300:: Pelvis
- 400a, 400b:: Obturator foramen
- 500, 600:: Puncture hole

## Claims

1. A puncture device, comprising:
a puncture needle having a hollow portion;
a needle tip portion positioned at a distal end of the puncture needle and configured to puncture a living body tissue; and
an elongated living body tissue supporting indwelling article accommodated in the hollow portion of the puncture needle and configured to be implanted into a living body to support the living body tissue.

2. The puncture device according to claim 1,
wherein the needle tip portion is removably held by or fixed to a distal end portion of the living body tissue supporting indwelling article.

3. The puncture device according to claim 1,
wherein the living body tissue supporting indwelling article includes:
a belt-like element configured to attach to a living body tissue;
a first string-like element connected at a proximal end thereof to a distal end of the belt-like element;
a second string-like element connected at a distal end thereof to a proximal end of the belt-like element;
and
a holding member connected to a proximal end of the second string-like element and removably held by a proximal end of the puncture needle;
the needle tip portion being removably held by or fixed to the distal end of the first string-like element.

4. The puncture device according to claim 2 or 3, wherein the needle tip portion has an anchoring effect of preventing the needle tip portion from returning to an opposite direction to a puncturing direction of the needle tip portion.

5. The puncture device according to claim 1,
wherein the needle tip portion is fixedly mounted at or integrated with a distal end portion of the puncture needle.

6. The puncture device according to any one of claims 1 to 5, wherein the puncture needle has a variable length.

7. The puncture device according to any one of claims 1 to 6, wherein the puncture needle has a flattened shape as viewed in a longitudinal direction thereof, and
the living body tissue supporting indwelling article has a belt-like portion which is attached to a living body tissue.

8. The puncture device according to any one of claims 1 to 7, wherein the puncture needle has a portion curved along a longitudinal direction thereof.

9. The puncture device according to claim 8,
wherein the puncture device is installed for rotational motion.

10. A puncture apparatus, comprising:
the puncture device according to claim 9;
a urethral-insertion member of a longitudinal shape configured to be inserted into a urethra; and
restriction means for restricting, when the puncture device is rotationally moved to puncture a living body tissue, a positional relationship between the puncture device and the urethral-insertion member such that the needle tip portion passes at a farther-position side from the center of rotational movement of the puncture device than the urethral-insertion member.

11. The puncture apparatus according to claim 10, further comprising
a vaginal insertion member of a longitudinal shape configured to be inserted into a vagina, wherein
the restriction means restricts a positional relationship between the puncture device and the vaginal insertion member such that, when the puncture device is rotationally moved to puncture a living body tissue, the needle tip portion does not collide with the vaginal insertion member.

12. The puncture apparatus according to claim 11, wherein the puncture device has a shaft portion which provides a rotational axis of the rotational moment, and
the restriction means includes a supporting member which supports the shaft portion for rotational movement and supports the urethral-insertion member and the vaginal insertion member.

13. The puncture apparatus according to claim 11 or 12, wherein at least one of the urethral-insertion member and the vaginal insertion member has provided thereon suction means capable of attracting a living body tissue thereto.

14. The puncture apparatus according to any one of claims 11 to 13, wherein the positional relationship between the urethral-insertion member and the vaginal insertion member is variable.
